# EUROPEAN PATENT APPLICATION

(11) **EP 4 268 721 A1**
(43) Date of publication of application: **01.11.2023**
(21) Application number: 20966563.7
(22) Date of filing: 25.12.2020
(51) Int. Cl.: A61B 5/22, A61B 5/00

(54) **MEDICAL DEVICE AND POSITION PROMPTING METHOD FOR ESOPHAGEAL MANOMETRY APPARATUS THEREOF**

(71) Applicant: Shenzhen Mindray Bio-Medical Electronics Co., Ltd., Shenzhen, Guangdong 518057 (CN)
(72) Inventor: PAN, Ruiling, Shenzhen, Guangdong 518057 (CN); LI, Lan, Shenzhen, Guangdong 518057 (CN); LIU, Jinglei, Shenzhen, Guangdong 518057 (CN); HUANG, Zhiwen, Shenzhen, Guangdong 518057 (CN); JIAO, Dongsheng, Shenzhen, Guangdong 518057 (CN)
(74) Representative: KIPA AB
(86) International application number: PCT/CN2020/139371
(87) International publication number: WO 2022/133998

(57) **Abstract**

The present invention provides a medical device and a position prompting method for an esophageal manometry apparatus thereof, the method comprising: detecting the position of a manometry apparatus moving in the esophagus, when detected that the manometry apparatus is in a target position range, outputting corresponding first prompt information, the fast prompt information being used to prompt that the manometry apparatus is in the target position range, thereby acquiring, after the manometry apparatus reaches the target position range, pressure data detected by the manometry apparatus, and using same as a target esophageal pressure. The present invention improves the working efficiency of doctors by assisting the doctors in determining whether a manometry apparatus is in place.

## Description

### TECHNICAL FIELD

The disclosure relates to medical fields, and more particularly to a medical device and position indication method for a pressure measurement apparatus of an esophageal pressure thereof.

### BACKGROUND

With the continuous development of mechanical ventilation technology, new mechanical ventilation strategies (such as lung recruitment, ventilation at prone position, etc.) have been applied in clinical practice, which improves re-expansion of collapsed alveolar, corrects ventilation/blood flow imbalance, improves oxygenation, and saves lives of many critically ill patients. However, positive pressure ventilation is originally opposite to negative pressure respiration in the physiological state of the human body, which can cause damage to the alveoli due to excessive positive end-expiratory pressure (PEEP). At the same time, low positive end-expiratory pressure cannot maintain end-expiratory alveolar dilation, making it difficult to improve blood and gas exchange. Therefore, it is urgently needed in clinical practice to calculate transpulmonary pressure (alveolar pressure -intrathoracic pressure) through dynamic monitoring of intrathoracic pressure, so as to provide personalized ventilation treatment for different patients. At present, esophageal pressure measurement is often used in clinical practice to reflect the intrathoracic pressure.

When measuring the esophageal pressure, the doctor needs to insert an esophageal pressure measurement tube with a balloon into stomach of the patient through a nasal cavity. The position of the balloon is one of the conditions to ensure accurate and effective measurement. In clinical practice, it is recommended to place the balloon lower one-third of the esophagus. When placing a pressure measurement tube, there is a high demand for operational experience of the doctor. For doctor with less experience, it may still be difficult to locate the optimal position of the balloon through multiple retractions and insertions of the pressure measurement tube. In clinical practice, determining whether the pressure measurement tube is in position currently relies heavily on the experience of doctor. For doctor with less experience, the work efficiency is low and it can cause pain to patient.

Therefore, in the process of positioning the esophageal pressure measurement tube, the work efficiency of doctor needs to be improved.

### SUMMARY

This disclosure mainly provides a medical device and a position indication method for a pressure measurement apparatus of an esophageal pressure thereof, aiming to improve the work efficiency of doctor.

An embodiment provides a position indication method for a pressure measurement apparatus of an esophageal pressure, including:
detecting a position of the pressure measurement apparatus which moves in an esophagus;
outputting first indication information when the pressure measurement apparatus is detected to be in a target position range, wherein the first indication information is configured to indicate that the pressure measurement apparatus is already in the target position range;
obtaining pressure data, which is detected by the pressure measurement apparatus which is already in the target position range, and using the pressure data as a target esophageal pressure.

An embodiment provides a medical device, including:
a pressure measurement apparatus, which is configured to detect a pressure in an esophagus, so as to obtain pressure data;
a detection device, which is configured to detect the pressure measurement apparatus which moves in the esophagus, so as to obtain detection data, which is related to a position of the pressure measurement apparatus;
a processor, which is configured to determine whether the pressure measurement apparatus is in a target position range according to the detection data, and to output first indication information when the pressure measurement apparatus is detected to be in the target position range, wherein the first indication information is configured to indicate that the pressure measurement apparatus is already in the target position range.

An embodiment provides a medical device, including:
a pressure measurement apparatus, which is configured to detect a pressure in an esophagus, so as to obtain pressure data;
a processor, which is configured to determine whether the pressure measurement apparatus is in a target position range according to the pressure data, and to output first indication information when the pressure measurement apparatus is detected to be in the target position range, wherein the first indication information is configured to indicate that the pressure measurement apparatus is already in the target position range.

An embodiment provides a position indication method for a pressure measurement apparatus of an esophageal pressure, including:
obtaining esophageal pressure data, which is measured by the pressure measurement apparatus, for at least one pressure change cycle;
obtaining airway pressure data for the at least one pressure change cycle;
displaying a pressure comparison view according to the esophageal pressure data and the airway pressure data, wherein the pressure comparison view includes a trend graph for the esophageal pressure and a trend graph for the airway pressure; and
further displaying, when the pressure comparison view is displayed, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; wherein the first target point of the esophageal pressure and the second target point of the esophageal pressure are two points for calculating a change of the esophageal pressure, and the first target point of the airway pressure and the second target point of the airway pressure are two points for calculating a change of the airway pressure.

An embodiment provides a medical device, including:
a processor, which is configured to:
obtain esophageal pressure data, which is measured by a pressure measurement apparatus, for at least one pressure change cycle;
obtain airway pressure data for the at least one pressure change cycle;
display a pressure comparison view according to the esophageal pressure data and the airway pressure data, wherein the pressure comparison view includes a trend graph for an esophageal pressure and a trend graph for an airway pressure; and
further display, when the pressure comparison view is displayed, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; wherein the first target point of the esophageal pressure and the second target point of the esophageal pressure are two points for calculating a change of the esophageal pressure, and the first target point of the airway pressure and the second target point of the airway pressure are two points for calculating a change of the airway pressure.

An embodiment provides a computer-readable storage medium which includes a program, wherein when the program is executed by a processor, the above method according to any embodiment of this disclosure is implemented.

According to the above embodiments, the medical device and position indication method for pressure measurement apparatus of esophageal pressure thereof, detect a position of the pressure measurement apparatus which moves in an esophagus, output first indication information when the pressure measurement apparatus is detected to be in a target position range, wherein the first indication information is configured to indicate that the pressure measurement apparatus is already in the target position range, such that pressure data, which is detected by the pressure measurement apparatus which is already in the target position range, can be obtained and used as a target esophageal pressure. This disclosure improves the work efficiency of doctor by assisting in determining whether the pressure measurement apparatus is in position.

### BRIEF DESCRIPTION OF THE DRAWINGS

FIG. 1 is a structural block diagram of an embodiment of a medical device provided by this disclosure.
FIG. 2 is a structural block diagram of an embodiment of a medical device provided by this disclosure.
FIG. 3 is a flowchart of an embodiment of a position indication method for a pressure measurement apparatus of an esophageal pressure provided by this disclosure.
FIG. 4 is a schematic diagram of an embodiment for outputting first indication information in a medical device provided by this disclosure.
FIG. 5 is a schematic diagram of an embodiment for outputting first indication information in a medical device provided by this disclosure.
FIG. 6 is a schematic diagram of an embodiment for outputting first indication information in a medical device provided by this disclosure.
FIG. 7 is a schematic diagram of an embodiment for outputting first indication information in a medical device provided by this disclosure.
FIG. 8 is a schematic diagram of an embodiment for outputting first indication information in a medical device provided by this disclosure.
FIG. 9A is a schematic diagram of a display interface when a pressure measurement apparatus is not in a target position range in a medical device provided by this disclosure.
FIG. 9B is a schematic diagram of a display interface when a pressure measurement apparatus is in a target position range in a medical device provided by this disclosure.
FIG. 10A is a schematic diagram of a display interface when a pressure measurement apparatus is not in a target position range in a medical device provided by this disclosure.
FIG. 10B is a schematic diagram of a display interface when a pressure measurement apparatus is in a target position range in a medical device provided by this disclosure.
FIG. 11 is a schematic diagram of a display interface when a pressure measurement apparatus is in a target position range in a medical device provided by this disclosure.
FIG. 12 is a schematic diagram of a display interface during expiration hold in a medical device provided by this disclosure.
FIG. 13 is a schematic diagram of a display interface of a medical device provided by this disclosure after expiration hold is completed.
FIG. 14 is a flowchart of an embodiment of a position indication method for a pressure measurement apparatus of an esophageal pressure provided by this disclosure.
FIG. 15 is a schematic diagram of an embodiment of a pressure comparison view in a medical device provided by this disclosure.
FIG. 16 is a schematic diagram of an embodiment of a pressure comparison view in a medical device provided by this disclosure.
FIG. 17 is a schematic diagram of an embodiment of a pressure comparison view in a medical device provided by this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, so as to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the characteristic, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are operable to distinguish different objects, rather than to describe a specific order. In addition, the terms "connection", and "linkage" mentioned in this disclosure include direct and indirect connection (linkage), unless otherwise specified.

This disclosure presents changes in a position of a pressure measurement tube during positioning the pressure measurement tube in esophagus through a visualized manner, which simplifies a workflow of doctor, improves an efficiency of tube placement, facilitates the clinical application of esophageal pressure and transpulmonary pressure, and provides effective support for the individualized application of mechanical ventilation therapy in clinical practice. The following is a detailed explanation through some embodiments.

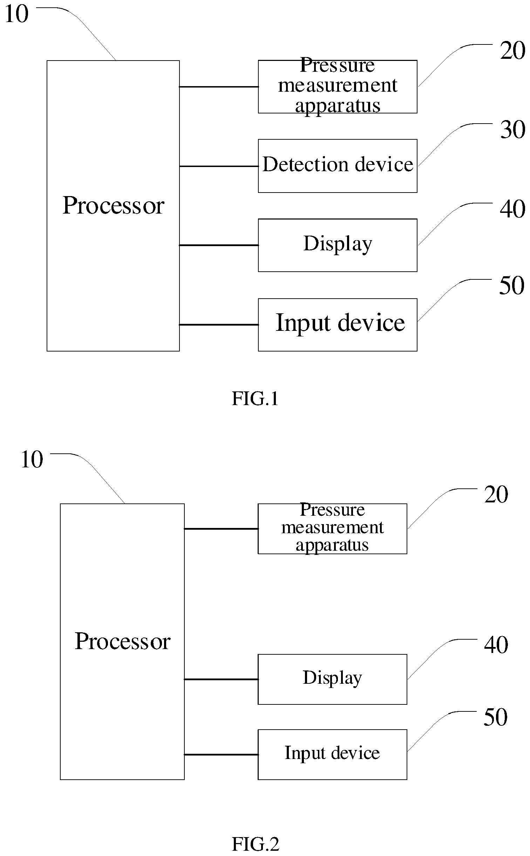

As shown in FIGS. 1 and 2, a medical device provided by this disclosure includes a processor 10 and a pressure measurement apparatus 20. The processor 10 is in communicational connection with the pressure measurement apparatus 20. The processor 10 is configured to control an operation of the medical device to achieve various functions of the medical device.

The pressure measurement apparatus 20 is configured to detect a pressure in an esophagus, so as to obtain pressure data. Conventional device for measuring esophageal pressure can be used, such as an esophageal pressure measurement tube with an balloon.

In the embodiment shown in FIG. 1, the medical device further includes a detection device 30, and the processor 10 is in communicational connection with the detection device 30. The detection device 30 is configured to detect the pressure measurement apparatus 20 which moves in the esophagus, so as to obtain detection data, which is related to a position of the pressure measurement apparatus. Of course, in the embodiment shown in FIG. 2, there may also be no detection device 30, and the processor 10 determines the position of the pressure measurement apparatus 20 according to the pressure data detected by the pressure measurement apparatus 20.

The processor 10 is configured to determine whether the pressure measurement apparatus 20 is in a target position range according to the detection data or pressure data. When the pressure measurement apparatus 20 is detected to be in the target position range, first indication information is outputted, which is configured to indicate that the pressure measurement apparatus 20 is already in the target position range. It can be seen that this disclosure improves the work efficiency of doctor by assisting in determining whether the pressure measurement apparatus 20 is in position. Usually, ventilators, anesthesia machines, etc., require the measurement of esophageal pressure during use, so the medical device can be either a ventilator or an anesthesia machine. Of course, the medical device can also be a medical device, which is configured to detect esophageal pressure.

In some embodiments, the medical device also includes a display 40 and an input device 50. The processor 10 is in communicational connection with the display 40 and input device 50. The input device 50 is configured to receive an input from a user (usually an operator), for example, one or more of a mouse, a keyboard, a touch display, a trackball, a joystick, or the likes, may be configured to receive an instruction inputted by a user, or the likes. User can perform input operations through the input device 50.

The display 40 is configured for outputting information, such as outputting visualization information. The display 40 can be a display that only has display function, or a touch display. It can be seen that, the display 40 and input device 50 are human-machine interaction devices for medical device, which can receive user inputted instructions and display visual information.

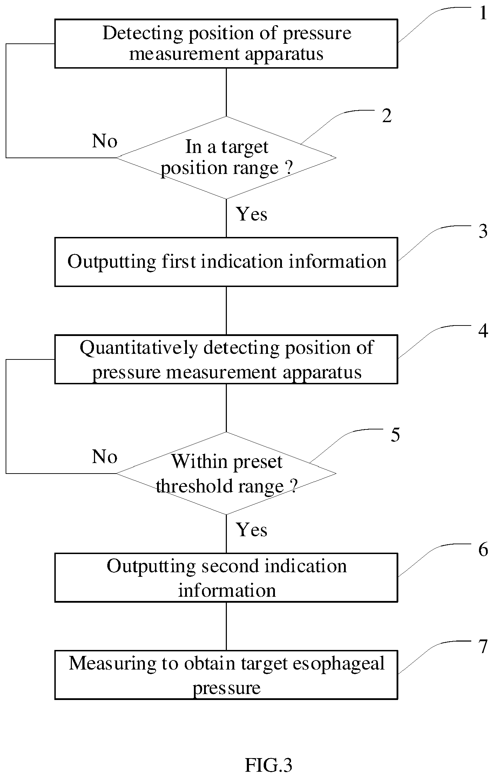

In the current process for detecting esophageal pressure, the doctor places an esophageal pressure measurement tube with an balloon into stomach of a patient and inflates the balloon. The doctor presses the abdomen of the patient to confirm the position of the pressure measurement tube. The doctor draws back the pressure measurement tube and determines according to the own experience whether it is in a target position range. If so, the pressure measurement tube is placed in the target position range and a pressure measured by the pressure measurement tube at this time is used as the esophageal pressure. By using the medical device provided by this disclosure, the doctor can issue instructions through the input device 50 before withdrawing the pressure measurement tube, causing the medical device to execute the process shown in FIG. 3. After receiving the first indication information, the pressure measurement tube can be considered as reaching the target position range, without relying on the personal judgment of the doctor, which is very convenient and saves the workload of the doctor.

Specifically, the process of the processor 10 for controlling the medical device to indicate the position of the pressure measurement apparatus 20 is shown in FIG. 3, including the following steps.

In step 1, a position of the pressure measurement apparatus which moves in an esophagus, is detected. For example, before withdrawing the pressure measurement tube, the doctor sends an instruction to start indication process through the input device 50, and the processor 10 responds to this instruction by detecting the position of the pressure measurement apparatus 20 which moves in the esophagus through the detection device 30 or the pressure measurement apparatus 20.

Specifically, in the embodiment shown in FIG. 1, the processor 10 responds to the instruction for starting the indication process and starts the detection device 30 to operate. The detection device 30 detects the pressure measurement apparatus 20 which moves in the esophagus (caused by the doctor through pulling back the pressure measurement apparatus 20), and obtains the detection data related to the position of the pressure measurement apparatus 20. Since the position of the pressure measurement apparatus 20 is mainly configured to determine whether it is in the target position range, the position of the pressure measurement apparatus 20 can be a relative distance between the pressure measurement apparatus 20 and the target position range.

The detection device 30 detects the pressure measurement apparatus 20 in real-time, and the corresponding detection data is real-time detection data, which can reflect the real-time position of the pressure measurement apparatus 20.

In the embodiment shown in FIG. 2, the processor 10 responds to the instruction for starting the indication process and starts the pressure measurement apparatus 20 to operate. The pressure measurement apparatus 20 detects the pressure in the esophagus and obtains the pressure data. The pressure measurement device 20 can detect the pressure in the esophagus in real-time, and the corresponding pressure data is real-time pressure data, which can reflect the real-time position of the pressure measurement device 20.

In step 2, the processor 10 determines whether the pressure measurement apparatus 20 is in the target position range according to the detection data or the pressure data. For example, in the embodiment shown in FIG. 1, the processor 10 obtains the detection data from the detection device 30, and then determines whether the pressure measurement apparatus 20 is in the target position range according to the detection data. If so, the process proceeds to step 3, otherwise, the position of the pressure measurement apparatus 20 is detected again, that is, the process returns to step 1, until determining that the pressure measurement apparatus 20 is in the target position range, that is, the process proceeds to step 3. In this embodiment, the processor 10 obtains real-time detection data, and by determining the real-time position of the pressure measurement apparatus 20, the processor 10 can quickly determine whether the pressure measurement apparatus 20 is in position (reaching the target position range).

The target location range can be pre-set in the medical device or set by the user. Usually, when detecting the esophageal pressure, the ideal measurement position of the pressure measurement apparatus 20 is lower one-third of the esophagus (divide the esophagus into three equal segments, the boundary point between the middle and lower segments is the lower one-third of the esophagus). However, this ideal position is difficult to determine, and in clinical practice, the position adjacent to this ideal position is usually used as the position for measuring the esophageal pressure by pressure measurement apparatus 20. Therefore, the target position range can be a position range that covers the lower one-third of the esophagus. The processor 10 also generates a score value according to the detection data or pressure data to reflect the position of the pressure measurement apparatus 20. Specifically, in this embodiment, the score value can be configured to reflect the relative distance between the pressure measurement apparatus 20 and the target position range. For example, the higher the score value, the closer the position of the pressure measurement apparatus 20 is to the target position range (the smaller the relative distance). A score threshold can be set. When the score value exceeds the score threshold, it indicates that the pressure measurement apparatus 20 is within the target position range. The score value after exceeding the score threshold is higher, the closer it is to the center position of the target position range (lower one-third of the esophagus).

The detection device 30 can be an image acquisition device, such as various cameras (such as endoscopes, etc.), which can be fixed with the pressure measurement apparatus 20 to capture images of the esophagus around the pressure measurement apparatus 20 (such as front, rear, etc.). Correspondingly, the detection data acquired by the image acquisition device includes image data, such as photos or videos. The processor 10 obtains image data from the image acquisition device, identifies the image data to detect the position of the pressure measurement apparatus 20, and generates a score value. For example, image recognition is performed on the image data to detect the position of the pressure measurement apparatus 20, and the score value is generated according to the relative distance between the position of pressure measurement apparatus 20 and the target position range. When the pressure measurement apparatus 20 is detected to be in the target position range, the process proceeds to step 3. For example, the image data is inputted into a pre-trained deep learning model, then the deep learning model outputs the corresponding score value and the determination result of whether the pressure measurement apparatus 20 is in the target position range. When the deep learning model outputs the result that the pressure measurement apparatus 20 is in the target position range, the process proceeds to step 3, etc.

The medical device can also include a sound generation apparatus, which is configured to send a sound signal (such as sound) at the pressure measurement apparatus 20, and the detection device 30 detects the sound signal to obtain the detection data. The processor 10 obtains the detection data from the detection device 30 and captures the position of the pressure measurement apparatus 20 according to the detection data. For example, the processor 10 processes the detection data to obtain a movement distance of the pressure measurement apparatus 20, and then determines the position of pressure measurement apparatus 20 according to a preset empirical value of the esophageal length, thereby generating a corresponding score value. When the pressure measurement apparatus 20 is detected to be in the target position range, the process proceeds to step 3. For example, when the pressure measurement apparatus 20 is inserted into the throat of the patient, it is calibrated by a doctor. When the pressure measurement apparatus 20 reaches the stomach, it is calibrated by the doctor. The length of the esophagus is determined through the two calibrations, and the movement distance of the pressure measurement apparatus 20 is obtained by processing the detection data. Comparing the movement distance with the length of the esophagus can determine the position of the pressure measurement apparatus 20 in the esophagus, and further determine the relative distance between the pressure measurement apparatus 20 and the target position range. Then the corresponding score value is generated.

The medical device can also include a light source, which is configured to send a light signal at the pressure measurement apparatus 20. The detection device 30 obtains the detection data by detecting the light signal, and the processor 10 obtains the detection data from the detection device 30, captures the position of the pressure measurement apparatus 20 according to the detection data. For example, the processor 10 processes the detection data to obtain the movement distance of pressure measurement apparatus 20, and then determines the position of the pressure measurement apparatus 20 according to the preset empirical value of esophageal length, thereby generating corresponding score value. When the pressure measurement apparatus 20 is detected to be in the target position range, the process proceeds to step 3. For example, when the pressure measurement apparatus 20 is inserted into the throat of the patient, it is calibrated by a doctor. When the pressure measurement apparatus 20 reaches the stomach, it is calibrated by the doctor. The length of the esophagus is determined through two calibrations, and the movement distance of the pressure measurement apparatus 20 is obtained by processing the detection data. Comparing the movement distance with the length of the esophagus can determine the position of the pressure measurement apparatus 20 in the esophagus, and further determine the relative distance between the pressure measurement apparatus 20 and the target position range. Then the corresponding score value is generated.

In the embodiment shown in FIG. 2, the processor 10 obtains the pressure data from the pressure measurement apparatus 20, and then determines whether the pressure measurement apparatus 20 is in the target position range according to the pressure data. If so, the process proceeds to step 3, otherwise, the position of the pressure measurement apparatus 20 is detected again, that is, the process returns to step 1, until determining that the pressure measurement apparatus 20 is in the target position range, that is, the process proceeds to step 3. In this embodiment, the processor 10 obtains real-time pressure data, and by determining the real-time position of the pressure measurement apparatus 20, the processor 10 can quickly determine whether the pressure measurement apparatus 20 is in position (reaching the target position range) and generate corresponding score value.

The processor 10 determines whether the pressure measurement apparatus 20 is in the target position range according to the pressure data. For example, during the dynamic change of the position of pressure measurement tube, the pressure data (pressure signal) acquired changes accordingly. When approaching the target position in the esophagus, the pressure signal has a certain pattern (at this time, the heart artifact is strong). Accordingly, whether the heart artifact of the pressure data exceeds the preset threshold, is determine. If so, it is determined that the pressure measurement apparatus 20 is in the target position range. Certain software algorithms can be configured to capture and match waveform characteristics to identify the position. Specifically, the processor 10 generates a waveform curve for the esophageal pressure according to the pressure data, detects a waveform characteristic of the waveform curve for the esophageal pressure, and determines that the pressure measurement apparatus 20 is in the target position range when the waveform characteristic detected is a preset characteristic, or generates a corresponding score value according to a difference between the detected waveform characteristic and the preset characteristic. The preset characteristic can be manually obtained by a doctor in advance. For example, after determining that the pressure measurement apparatus 20 is located in the stomach, the doctor withdraws the pressure measurement apparatus 20 and acquires the pressure data detected by the pressure measurement apparatus 20. After the doctor determines that the pressure measurement apparatus 20 reaches the target position range, the doctor calibrates it. At least the pressure data obtained during this period is configured to generate the waveform curve for the esophageal pressure, and the preset characteristic can be obtained according to the waveform curve for the esophageal pressure. The above operations can be repeated to obtain a large number of preset characteristics, and after synthesis (such as averaging), a final preset characteristic can be obtained.

In step 3, when detecting that the pressure measurement apparatus 20 is in the target position range, the processor 10 outputs first indication information. For example, the display 40, which is connected with the processor 10, displays at least one of the following indication information: text indication information, graphical indication information, indication information for displaying picture(s) or video(s), sound indication information, and light indication information. Through the first indication information, the doctor can know that the pressure measurement apparatus 20 is already in the target position range, which is very convenient.

In order to present the position of the pressure measurement apparatus 20 in the esophagus more intuitively, the processor 10 displays an interface element for displaying the position of the pressure measurement apparatus 20 through the display 40, which includes the first indication information. That is, from the moment the doctor withdraws the pressure measurement tube until the pressure measurement tube is located in the target position range, the doctor can visually see the position of the pressure measurement apparatus 20 through the interface element. Combined with the first indication information, the doctor can quickly withdraw the pressure measurement apparatus 20 to the target position range, improving the work efficiency. The interface element and the first indication information can be presented in various ways, and the following examples are provided in conjunction with the attached drawings.

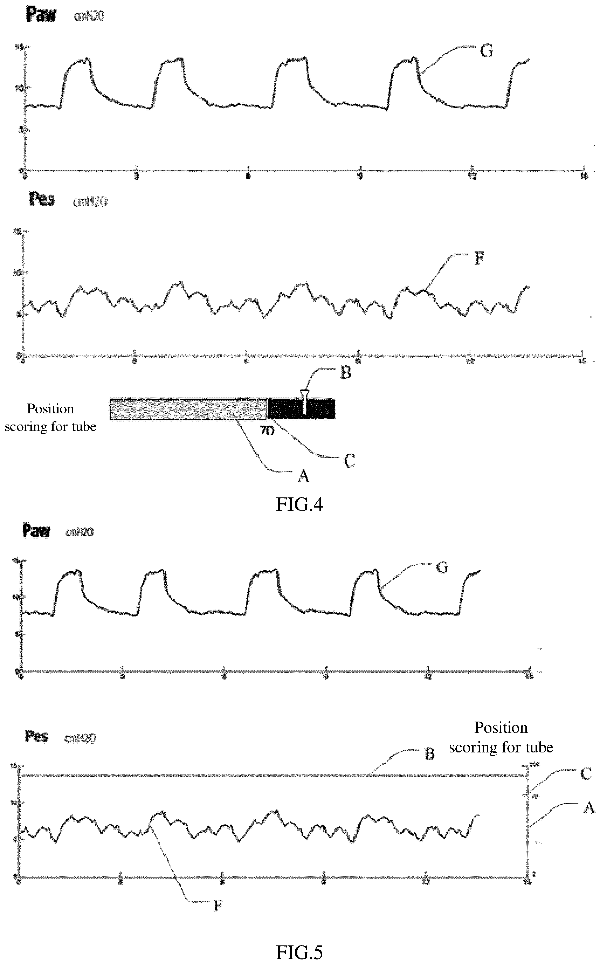

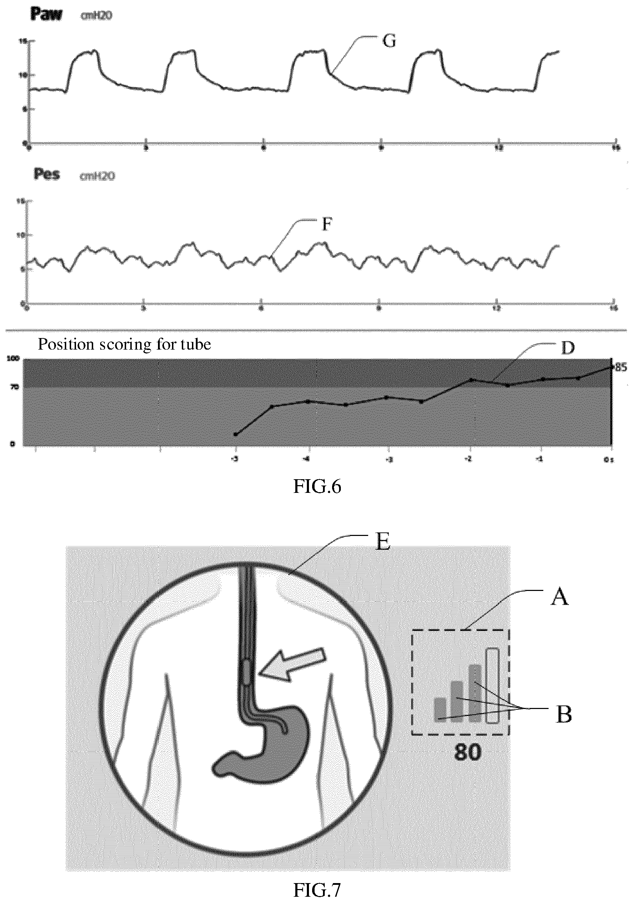

Please refer to FIG. 4- FIG. 10B. The interface element includes the score value mentioned above, and the processor 10 displays the score value on the display 40 which is connected with the processor. The score value in step 2 is generated according to real-time detection data or pressure data, so the score value displayed on display 40 is also real-time. The processor 10 also performs Differential display on the score value corresponding to the pressure measurement apparatus when it is in the target position range. The Differential display can display the score value in other colors, in bold, highlighted, marked, and other forms. This disclosure is not limited. The score value can be directly displayed in numerical form, as shown in FIG. 7, FIG. 9A/B, and FIG. 10A/B, or in the form of a mark B in a measurement value bar A, as shown in FIG. 4, FIG. 5, FIG. 7- FIG. 10B, or in the form of a broken line D in a coordinate, as shown in FIG. 6. The score value can be a type of first indication information, that is, the doctor can determine whether the pressure measurement apparatus 20 is in the target position range, or close to the target position range by comparing the score value with the predetermined score threshold. Of course, this process can also be completed by the processor 10, which is explained as an example in this embodiment.

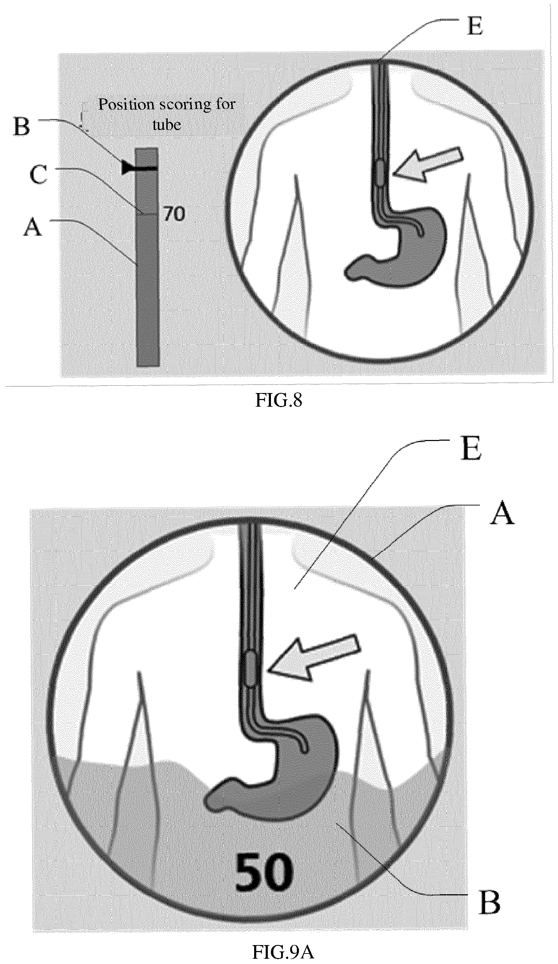

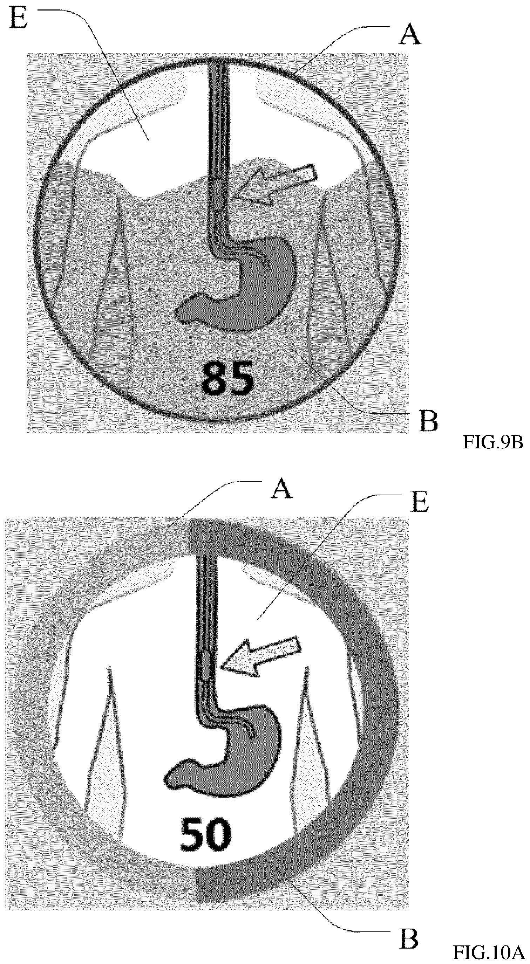

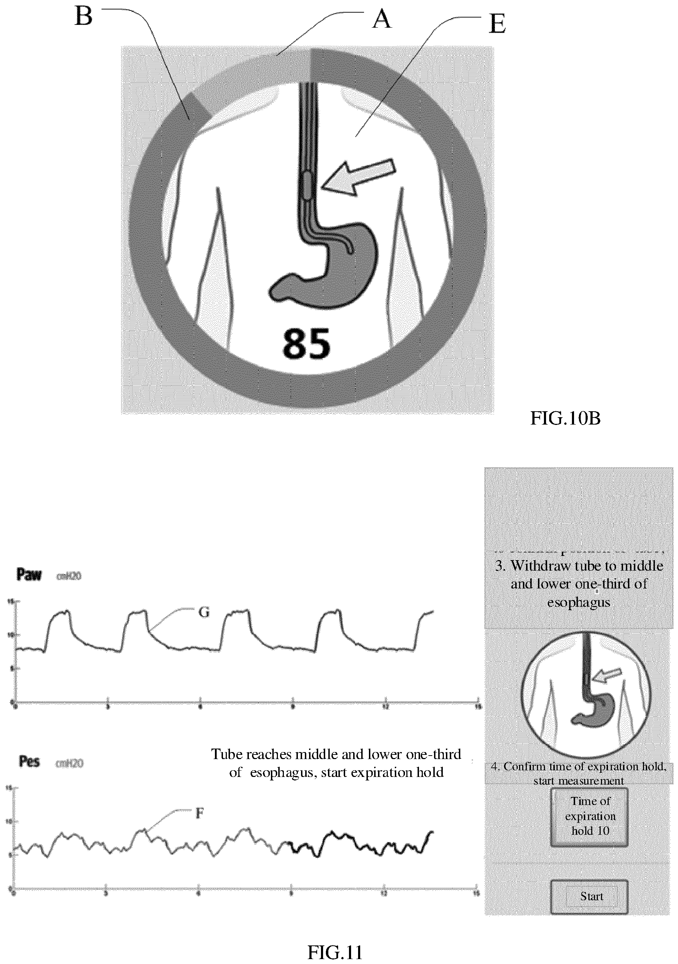

In the embodiments shown in FIGS. 4, 5, and 7 to 10B, the interface element also includes a measurement value bar A. The processor 10 also displays the measurement value bar A of the score value on the display 40 and marks the position of the score value on the measurement value bar A. The measurement value bar A reflects the range of the score value, which can take many forms, such as the straight bar in FIG. 4, the coordinate in FIG. 5, the progress bar in FIG. 7, the annular bar in FIG. 9A/B, and the circle in FIG. 10A/B.

A first mark B is set on the measurement value bar A, to mark the position of the score value. The interface element can also include the first mark B. The first mark B can be the color filled on the measurement value bar A, as shown in gray in FIG. 7, FIG. 9A/B, and FIG. 10A/B. The first mark B can also be various shapes and patterns, as shown in FIGS. 4, 5, and 8. Due to the fact that the combination of the first mark B and the measurement value bar A can reflect real-time score values, the first indication information can also include the first mark B and/or the measurement value bar A. In FIGS. 7, 9A/B, and 10A/B, if the score value does not exceed the score threshold, the first mark B fills the corresponding area of the measurement value bar A with a color (such as gray). If the score value exceeds the score threshold, the first mark B fills the corresponding area of the measurement value bar A with another color (such as green). This is equivalent to using a color to represent the score threshold. In this way, the doctor can distinguish whether the pressure measurement apparatus 20 is in the target position range through the first mark B. In FIGS. 4, 5, and 8, the score threshold (70 in the figure) is also displayed on or adjacent to the measurement value bar A, and the score threshold is marked with a second mark C on the measurement value bar A. The first indication information can also include at least one of the measurement value bar A, score value, first mark B, and second mark C. The measurement value bar A, the first mark B, and the second mark C have various forms, as shown in FIGS. 4 and 5. In FIG. 5, the measurement value bar A is a vertical axis, the first mark B is a straight line for the score value, and the second mark C is the scale of the score threshold on the vertical axis. It can be seen that the doctor can determine at a glance whether the pressure measurement apparatus 20 is in the target position range or close to the target position range through the mark on the measurement value bar A and the measurement value bar A. It is very convenient and does not require personal judgment.

In the embodiment shown in FIG. 6, the interface element includes a trend graph for the change of the score value. The processor 10 displays the trend graph for the change of the score value on the display 40. The trend graph for the change can be a curve graph, a broken line graph, a scatter graph, etc., which can reflect the trend of change of the score value. This disclosure is not limited to this. The processor 10 marks a preset qualified range on the trend graph for change , as shown in the dark area in FIG. 6. The score values within the qualified range are the corresponding score values when the pressure measurement apparatus 20 is in the target position range. In this embodiment, the trend graph for change is illustrated with the broken line graph as an example, which includes the broken line D indicating the change of the score value. That is, the doctor can determine that the pressure measurement apparatus 20 is in the target position range by seeing that the broken line D enters the dark area in FIG. 6.

In the embodiments shown in FIGS. 7-10B, the processor 10 also displays a schematic diagram E for placing the tube on the display 40, which is configured to indicate the doctor to place the pressure measurement tube. As shown, the placement diagram E shows the esophagus, stomach, and balloon, and a third mark (as shown by the arrow in the figure) is set at the target position of the esophagus (lower one-third of the esophagus). The position of the balloon in the esophagus as shown in schematic diagram E can reflect the true position of the pressure measurement tube. Of course, the balloon in schematic diagram E can also be fixed at the target position, reminding the doctor to withdraw the balloon to the target position. In this way, doctor can perform the tube placement operation better through the schematic diagram E for placing the tube.

In the embodiments shown in FIGS. 9 (A/B) and 10 (A/B), the measurement value bar A is displayed in combination with the schematic diagram E for placing the tube. For example, in FIG. 9 (A/B), the measurement value bar A overlaps the schematic diagram E for placing the tube. For example, in FIG. 10 (A/B), both the measurement value bar A and the schematic diagram E for placing the tube are circular, and the measurement value bar A is set on the periphery of the schematic diagram E for placing the tube. In this way, it can effectively save the display area of the interface element.

As shown in FIGS. 4, 5, 6 and 11, the processor 10 generates a trend graph for the esophageal pressure Pes according to the pressure data obtained from the pressure measurement apparatus 20 and displays it through the display 40, which is connected with the processor. The trend graph for the esophageal pressure Pes can use scatter graph, curve graph, broken line graph, column graph, etc., which can reflect the trend of change of the esophageal pressure over time. This disclosure is not limited to this. In this embodiment, the trend graph for the esophageal pressure Pes includes waveform curve for the esophageal pressure F and the corresponding horizontal coordinates and vertical coordinates. Displaying the waveform curve for the esophageal pressure F is beneficial for doctor to understand the current esophageal pressure situation of the patient and can also assist in determining whether the pressure measurement tube is in position.

In order to output the first indication information, the processor 10 can be further configured to differentially display, in the trend graph for the esophageal pressure Pes, the corresponding region when the pressure measurement apparatus is already in the target position range. For example, in the waveform curve for the esophageal pressure F, the waveform curve segment corresponding to the pressure measurement apparatus 20 in the target position range is displayed differentially. Differential display can be achieved by displaying the waveform curve segment in other colors, in bold, highlighted, marked, or other forms, without any limitations in this disclosure. As shown in FIG. 11, the bold section of the waveform curve for the esophageal pressure F is the corresponding waveform curve segment when the pressure measurement apparatus 20 is in the target position range. From this, doctor can also determine whether the pressure measurement apparatus 20 is in the target position range while checking the waveform curve for the esophageal pressure F, which is very convenient.

The processor 10 also obtains airway pressure data, for example, through a pressure sensor, which can be placed in a host or inside the body of the patient. For example, the pressure sensor is inserted into the body of the patient through a pressure measurement tube, for example, placed in the nasal cavity of the patient to obtain pressure data. Optionally, for example, the pressure sensor is placed in the host and other components transmit the airway pressure to the host. Thus, the pressure data is detected and processed to obtain airway pressure data. The pressure sensor can be a pressure sensor of the medical device or an external pressure sensor. The processor 10 generates the trend graph for the airway pressure Paw according to the airway pressure data, and displays it through the display 40, which is connected with the processor. The trend graph for the airway pressure Paw can use scatter graph, curve graph, broken line graph, column graph, etc., which can reflect the trend of change of airway pressure over time. This disclosure is not limited to this. In this embodiment, the trend graph for the airway pressure Paw includes a waveform curve for the airway pressure G and the corresponding horizontal coordinates and vertical coordinates. Displaying the waveform curve for the airway pressure G is beneficial for doctor to understand the current airway pressure situation of the patient and facilitate the withdrawal of the pressure measurement tube.

Similarly, in order to output the first indication information, the processor 10 can be further configured to differentially display, in the trend graph for the airway pressure Paw, the corresponding region when the pressure measurement apparatus is already in the target position range. For example, in the waveform curve for the airway pressure G, the waveform curve segment corresponding to the pressure measurement apparatus 20 in the target position range is displayed differentially. From this, doctor can also determine whether the pressure measurement apparatus 20 is in the target position range while checking the waveform curve for the airway pressure G, which is very convenient.

As shown in FIG. 11, the processor 10 further displays corresponding indication information for doctor operation on display 40, when the pressure measurement apparatus 20 is moving to the target position range. That is, the processor 10 further displays corresponding indication information for doctor operation on display 40, when the processor 10 cooperates with the detection device or pressure measurement apparatus to detect the position of the pressure measurement apparatus which moves in the esophagus. The indication information for doctor operation can include video, animation, sound, text, illustration, color, and other indication information. The indication information for doctor operation can include: 1. indicating the doctor to confirm that the tube is inserted into the stomach and inflated; 2. pressing the abdomen of the patient to confirm the position of the tube; 3. withdrawing the tube to the middle and lower one-third of the esophagus. After the pressure measurement apparatus 20 is in the target position range, the indication information for doctor operation also includes information for indicating the doctor to proceed next operation step, as shown in FIG. 11, "The tube has reached the lower one-third of the esophagus, please start exhaling and maintaining.

In summary, when the doctor withdraws the pressure measurement tube, the display can display the dynamic change process of the position of the pressure measurement tube relative to the target position range in real time, so as to guide the doctor to adjust the position of the pressure measurement tube, and give the doctor clear and conclusive indications through the first indication information, which is very convenient.

Doctor can determine that the pressure measurement apparatus is in the target position range according to the above steps, and then the process proceeds to step 7, for obtaining the pressure data detected by the pressure measurement apparatus in the target position range and using it as the target esophageal pressure. Of course, doctor can also perform the entire process shown in FIG. 3. Considering the accuracy of processor 10 in determining the position of the pressure measurement apparatus, in this embodiment, steps 1-3 above are used as qualitative determination for the position of the pressure measurement apparatus. Below, quantitative determination for the position of the pressure measurement apparatus is made to ensure that it is accurately in the target position range. The specific process is shown in the following steps.

In step 4, the processor 10 quantitatively detects the position of the pressure measurement apparatus 20 which is static in the target position range, and obtains the quantitative value. This process is equivalent to the static quantitative determination stage of the position of the pressure measurement apparatus 20. The commonly used occlusion test in clinical practice can be used. During the test, the position of the pressure measurement tube is kept unchanged, and the ventilator is set to end-expiratory occlusion (expiration hold) for a period of time, and an instruction to start the expiration hold is inputted through the input device 50, so as to observe inspiratory effort of the patient (for patient with strong spontaneous respiration) or gently press chest wall of the patient (for patient without spontaneous respiration). After the expiration hold is completed, an instruction to stop the expiration hold is inputted through the input device 50. The processor 10 obtains the pressure data measured during the expiration hold from the pressure measurement apparatus 20, that is, the pressure data measured by the pressure measurement apparatus 20 during the period between receiving the instruction to start the expiration hold and receiving the instruction to stop the expiration hold. The processor 10 also obtains the airway pressure data during the expiration hold, such as obtaining the airway pressure data through a pressure sensor during the period between receiving the instruction to start the expiration hold and receiving the instruction to stop the expiration hold. The pressure sensor can be placed on the host or in the body of the patient, for example, the pressure sensor is inserted into the body of the patient through a pressure measurement tube, such as, placed in the nasal cavity of the patient, to obtain the pressure data. For example, a pressure sensor is placed in the host, and other components transmit the pressure in the airway to the host, thereby detecting the pressure data, and processing the pressure data to obtain the airway pressure data. The processor 10 processes the airway pressure data during the expiration hold into a waveform curve for the airway pressure, identifies start point and peak point of change of pressure from the waveform curve for the airway pressure, and calculates the change of pressure (pressure difference) Δ Paw between the start point and peak point. Similarly, the processor 10 processes the esophageal pressure data during the expiration hold into a waveform curve for the esophageal pressure, identifies start point and peak point of change of pressure from the waveform curve for the esophageal pressure, and calculates the change of pressure (pressure difference) Δ Pes between the start point and peak point. Then the processor 10 further calculates a ratio Δ Paw/Δ Pes between the change of airway pressure Δ Paw and the change of esophageal pressure Δ Pes, during the expiration hold, wherein such ratio is the quantitative value.

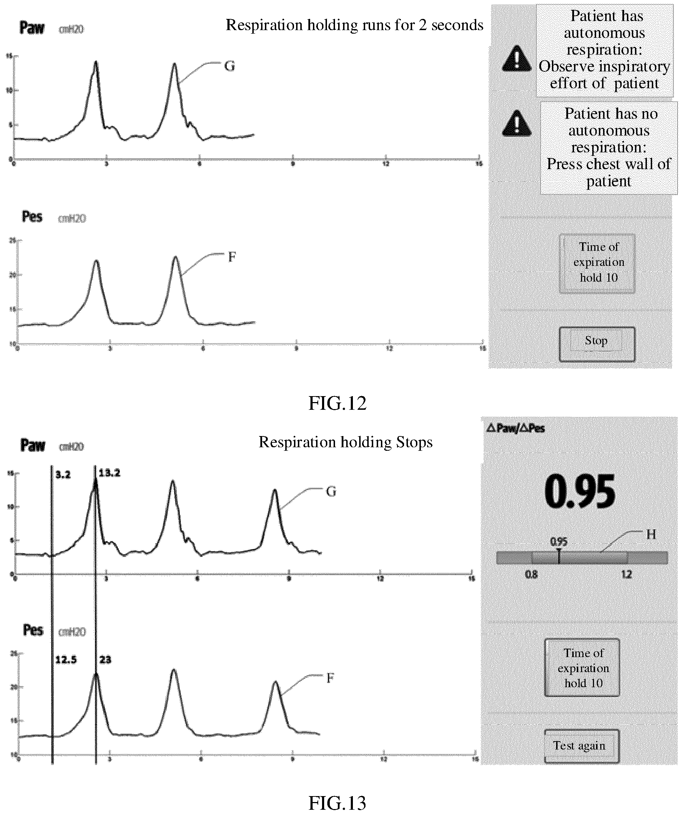

When quantitatively detecting the position of the static pressure measurement apparatus in the target position range, the processor 10 also displays, on the display, the corresponding indication information for doctor operation. The indication information for doctor operation can include video, animation, sound, text, illustration, color, and other indication information. As shown in FIG. 11, the indication information for doctor operation can include: after confirming time period for the expiration hold, start measuring. At the latest, after the processor 10 outputs the first indication information, the processor 10 displays a virtual button (such as the "Start" button in FIG. 11) on the display interface of the display to enable the expiration hold, and displays the time period recommended for the expiration hold (the time period recommended for the expiration hold shown in FIG. 11 is 10 seconds). Therefore, regardless of the experience of the doctor, according to the above indications, corresponding operations can be completed, improving the work efficiency. The doctor operates the input device by clicking on the virtual button to start the expiration hold, that is, the doctor sends the instruction to start the expiration hold. The processor 10 also displays a virtual button on the display interface of the display to stop the expiration hold (as shown in the "Stop" button in FIG. 12). The doctor operate the input device by clicking on the virtual button to stop the expiration hold, that is, the doctor sends the instruction to stop the expiration hold. Of course, in some embodiments, the instruction to stop the expiration hold can also be automatically triggered. For example, a recommended time period for expiration hold is pre-set, and the timing starts after the expiration hold starts. After the recommended time period for expiration hold, the instruction to stop the expiration hold is automatically determined. The recommended time period for exhalation retention can be set by the system or by a doctor.

As shown in FIGS. 12 and 13, after receiving the instruction to start the expiration hold, the processor 10 displays the current expiration hold state on the display interface of the display, as well as real-time waveform curve for the airway pressure G and/or waveform curve for the esophageal pressure F. The waveform curve for the airway pressure G and waveform curve for the esophageal pressure F can be displayed separately or in the same coordinate. During the expiration hold, the current state of expiration hold is a time period that the current expiration hold runs for, as shown in "Expiration holding runs for 2 seconds" in FIG. 12. After the expiration hold is completed, the current state of the expiration hold is that the expiration hold stops, as shown in FIG. 13.

As shown in FIG. 13, after receiving the instruction to stop the expiration hold, the processor 10 displays the quantitative value (0.95 in the figure) and threshold range (0.8-1.2 in the figure) on the display. In this embodiment, the measurement value bar H within the threshold range is displayed, and the position of the quantitative value is marked in the measurement value bar H.

In step 5, the processor 10 determines whether the quantitative value is within a preset threshold range. If so, the process proceeds to step 6, otherwise, the process returns to step 4. Of course, if the quantitative value is not within the preset threshold range, the process returns to step 1. For example, if the quantitative value is not within the preset threshold range, it may be that the respiration is not held properly, and the doctor can be indicated on the display to perform the quantitative testing again. It may also indicate that the determination that the pressure measurement apparatus is in the target position range in steps 1-3 is not accurate, and the doctor can be indicated on the display to repeat the process in FIG. 3.

The threshold range can be set as needed. In this embodiment, the threshold range is 0.8-1.2, which means that the ratio ΔPaw/Δ Pes is within the range of 0.8-1.2, indicating that the pressure measurement apparatus is indeed in the target position range, otherwise it is not in the target position range. Of course, in some embodiments, the doctor can also determine whether the quantitative value is within the preset threshold range. If so, the doctor sends a confirmation instruction through the input device, and the processor 10 responds to the confirmation instruction and executes step 7. Medical devices also provide a freezing function for the waveform curve for the airway pressure G and/or the waveform curve for the esophageal pressure F. During the expiration hold, the processor receives the freezing instruction inputted by the doctor, and freezes the waveform curve for the airway pressure G and/or waveform curve for the esophageal pressure F and saves them. It can help the doctor to customize and calculate the quantitative value on the frozen waveform, and display historical frozen waveforms on the display interface when triggered by a review instruction.

The above process shows that during the quantitative detection, the display screen displays the real-time waveforms for the airway pressure and esophageal pressure, operation indication, indication for time duration of the expiration hold, reference standard for successful positioning (threshold range), calculation result (quantitative value) for positioning, etc., providing comprehensive guidance on how doctor operates and makes determination, improving the work efficiency.

In step 6, when the quantitative value is within the preset threshold range, the processor 10 outputs second indication information. For example, the display 40, which is connected with the processor 10, displays at least one of the following indication information: text indication information, graphical indication information, indication information for displaying picture or video, sound indication information, and light indication information. The second indication information can include the quantitative value, threshold range, etc., mentioned above. Through the second indication information, the doctor can quantitatively determine that the pressure measurement apparatus 20 is already in the target position range, which is very convenient.

In step 7, the processor 10 obtains the pressure data detected by the pressure measurement apparatus in the target position range and uses it as the target esophageal pressure, as both the qualitative determination in step 2 and the quantitative determination in step 5 indicate that the pressure measurement apparatus is within the target position range, its credibility is very high. From this, the measurement of esophageal pressure is completed. It can be seen that the above measurement process of esophageal pressure has a high degree of automation. Doctor only needs to focus on placing the pressure measurement tube. Through various indications displayed on the display, doctor can quickly and accurately places the pressure measurement tube at lower one-third of the esophagus, reducing discomfort of the patient and improving the work efficiency of the doctor.

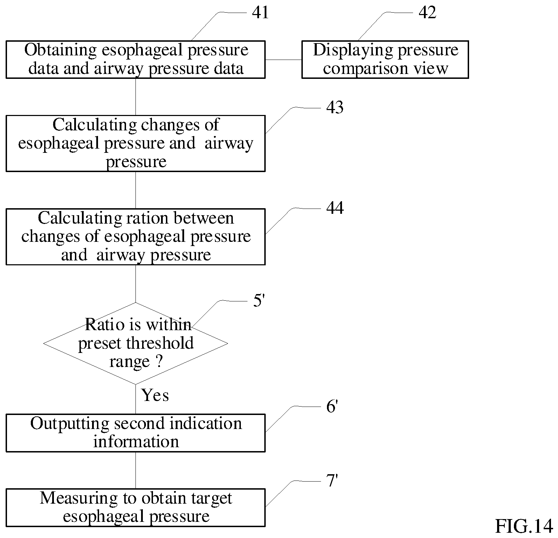

The medical device provided by this disclosure can also adopt another position indication method, as shown in FIG. 14, including the following steps.

In step 41, esophageal pressure data and airway pressure data are obtained. Specifically, the processor 10 obtains the esophageal pressure data, which is measured by a pressure measurement apparatus 20, for at least one pressure change cycle. The processor 10 also obtains the airway pressure data for the at least one pressure change cycle, such as obtaining the airway pressure data through a pressure sensor, which can be placed in the host or in the body of the patient. For example, the pressure sensor is inserted into the body of the patient through a pressure measurement tube, for example, placed in the nasal cavity of the patient to obtain pressure data. For example, the pressure sensor is placed in the host, and the pressure in the airway is transmitted to the host by other components to detect the pressure data. That is, the processor 10 obtains the esophageal pressure data and airway pressure data measured from a patient during the same time period, during which the patient spontaneously breathes or is pressed, resulting in changes of the esophageal pressure and airway pressure. This time period includes at least one pressure change cycle.

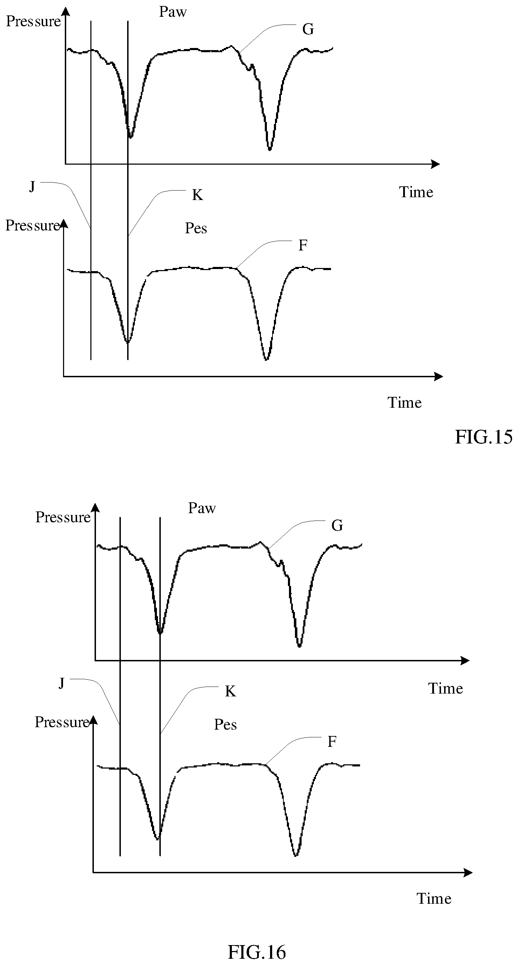

In an application scenario, after the doctor withdraws the pressure measurement apparatus 20 back to the target position range, whether it is subjective determined that the pressure measurement apparatus 20 is located in the target position range or qualitatively determined that the pressure measurement apparatus 20 is located in the target position range, a quantitative determination needs to be made on the position of the pressure measurement apparatus 20 to confirm that it is truly located in the target position range. Doctor can perform a Baydur Occlusion Test on a ventilator to make quantitative determination. The principle is to perform end-expiratory occlusion and calculate the ratio of change of the esophageal pressure to change of the airway pressure during the occlusion period. When the patient has autonomous respiration, the waveforms of airway pressure and esophageal pressure is shown in FIG. 15, and both decrease simultaneously, that is, the ratio of decreased amplitude of the esophageal pressure to decreased amplitude of the airway pressure is calculated. When the patient has no autonomous respiration, the doctor needs to gently press the chest wall of the patient during the occlusion period. At this time, the waveform is shown in FIG. 13, and the airway pressure and the esophageal pressure increase simultaneously, that is, the ratio of increased amplitude of the esophageal pressure to increased amplitude of the airway pressure is calculated.

Specifically, during the testing period, the pressure measurement apparatus 20 is kept in position, the ventilator is set to end-expiratory occlusion (expiration hold) for a period of time, and an instruction to start the expiration hold is inputted through the input device 50 to observe inspiratory effort of the patient (for patient with strong spontaneous respiration) or gently press chest wall of the patient (for patient without spontaneous respiration). After the expiration hold is completed, an instruction to stop the expiration hold is inputted through the input device 50. The processor 10 obtains the pressure data measured during the expiration hold from the pressure measurement apparatus 20, that is, the pressure data measured by the pressure measurement apparatus 20 during the period between receiving the instruction to start the expiration hold and receiving the instruction to stop the expiration hold. This pressure data is the esophageal pressure data for at least one pressure change cycle. The processor 10 also obtains the airway pressure data during the expiration hold, such as obtaining the pressure data measured by a pressure sensor placed in the nasal cavity of the patient during the period between receiving the instruction to start the expiration hold and receiving the instruction to stop the expiration hold. The pressure data is processed to obtain the airway pressure data for the at least one pressure change cycle.

In step 42, the processor 10 processes the esophageal pressure data and airway pressure data to obtain a pressure comparison view, which is displayed on the display 40. As shown in FIG. 13 and FIG. 15, the pressure comparison view includes a trend graph for the esophageal pressure Pes and trend graph for the airway pressure Paw. The trend graph is also called transition graph, running graph, chain graph, tendency graph, etc. The trend graph can be configured to reflect the relationship between one or more variables and time, that is, the development trend of one or more variables over time. For example, the trend graph can take time as the horizontal axis and the variable(s) to be observed as the vertical axis to observe the trend and/or deviation of the change or development of the variable(s). The observed variable(s) on the vertical axis can be absolute quantities/absolute values, mean values, incidence rates, etc. In this embodiment, time on the horizontal axes of the trend graph for the esophageal pressure and the trend graph for the airway pressure can be millisecond, second, etc., and the vertical axis is pressure values of the corresponding esophageal pressure and airway pressure. That is, the trend graph for the esophageal pressure is configured to reflect the trend of the esophageal pressure over time, and the trend graph for the airway pressure is configured to reflect the trend of the airway pressure over time. The trend graph for the esophageal pressure Pes and the trend graph for the airway pressure Paw can be displayed in the same coordinate system, that is, they can share the horizontal and vertical coordinates, or they can be displayed separately as shown in FIGS. 13 and 15. The latter one is explained as an example. As shown in FIGS. 13 and 15, the trend graph for the esophageal pressure Pes and the trend graph for the airway pressure Paw are set perpendicularly, and their time coordinates are the same (aligned in time).

The trend graph for the esophageal pressure includes a curve graph, a scatter graph, a broken line graph, a histogram, a bar graph, a box plot or a combination thereof. The trend graph for the airway pressure includes a curve graph, a scatter graph, a broken line graph, a histogram, a bar graph, a box plot or a combination thereof. They can reflect the changes in the esophageal pressure and airway pressure. This embodiment takes a curve graph as an example to illustrate. The curve graph for the esophageal pressure includes waveform curve for the esophageal pressure F, and the curve graph for the airway pressure includes waveform curve for the airway pressure G.

When processor 10 displays the pressure comparison view on the display interface of a monitor, it also displays a time point and/or a pressure value of a first target point of the esophageal pressure, time point and/or a pressure value of a second target point of the esophageal pressure, a time point and/or a pressure value of a first target point of the airway pressure, a time point and/or a pressure value of a second target point of the airway pressure. When displaying the various time points, pressure values, etc., they can be displayed in the form of text (such as numbers), making it clear to users at a glance. Optionally, the target point can be marked on the waveform curve, and the user can also know the time point and pressure value by combining the coordinates. In this embodiment, as shown in FIG. 13, the first and second target points of the esophageal pressure are marked on the waveform curve for the esophageal pressure F, and the pressure values of the first and second target points of the esophageal pressure (12.5 and 23 in the figure) are displayed, meanwhile the first and second target points of the airway pressure are also marked on the waveform curve for the airway pressure G, and the pressure values of the first and second target points of the airway pressure (3.2 and 13.2 in the figure) are also displayed. The processor 10 can automatically mark the first and second target points, and the user can also adjust the positions of the marks of the first and second target points, thereby manually setting the first and second target points. Among them, the first target point of the esophageal pressure and the second target point of the esophageal pressure are the two points for calculating the change in esophageal pressure, while the first target point of the airway pressure and the second target point of the airway pressure are the two points for calculating the change of the airway pressure. From this, the user can calculate the ratio of the change of the esophageal pressure to the change of the airway pressure by comparing the information displayed in the pressure comparison view, and then determine whether the pressure measurement apparatus 20 is in the target position range.

Specifically, the first target point of the esophageal pressure is a start point at which the pressure begins to change (increase or decrease) during a change cycle in curve F. The start point can be calculated through a preset algorithm, or can be other points near the start point within a start range, such as the points in the relatively flat section of curve F in FIGS. 13 and 15. That is to say, there are many points that can be selected as the start point. Similarly, the first target point of the airway pressure is the start point for the pressure to change (increase or decrease) during a change cycle in curve G. The start point can be calculated through a preset algorithm, or can be other points near the start point within the start range, such as the points in the relatively flat section of curve G in FIGS. 13 and 15. That is to say, there are many points that can be selected as the start point.

The second target point of the esophageal pressure is a peak point of the change (increase or decrease) of the pressure during a change cycle in curve F. The strictly defined peak point can be used as the second target point of the esophageal pressure, or other points near the peak point can be used as the second target point of the esophageal pressure as if they are within the peak range, such as the points near the highest or lowest point in curve F in FIG. 13 and FIG. 15. That is to say, there are many points that can be selected as the peak point. The second target point of the esophageal pressure and the first target point of the esophageal pressure can be in the same change cycle or in different change cycles. Similarly, the second target point of the airway pressure is the peak value of the change (increase or decrease) of the pressure during a change cycle in curve G. The strictly defined peak point can be used as the second target point of the airway pressure, and other points near the peak point can also be used as the second target point of the airway pressure as if they are within the peak range, such as the points near the highest or lowest point in curve G in FIGS. 13 and 15. That is to say, there are many points that can be selected as the peak point. The second target point of the airway pressure and the first target point of the airway pressure can be in the same change cycle or in different change cycles.

The processor 10 marks the first and second target points of the esophageal pressure on the waveform curve for the esophageal pressure F, and the first and second target points of the airway pressure on the waveform curve for the airway pressure G, in various ways. In FIGS. 13 and 15, as the time coordinates of the waveform curve for the esophageal pressure F and the waveform curve for the airway pressure G are the same, the waveform curve for the esophageal pressure F and the waveform curve for the airway pressure G are set perpendicularly. Therefore, the first target point of the esophageal pressure and the first target point of the airway pressure can be marked simultaneously through a first cursor line J, and the second target point of the esophageal pressure and the second target point of the airway pressure can be marked simultaneously through a second cursor line K. In this way, the first target point of the esophageal pressure and the first target point of the airway pressure are at the same time, and the second target point of the esophageal pressure and airway pressure are at the same time, which is beneficial for the user to associate and compare the two curves and various target points. The user adjusts (for example, dragging) the positions of the first and second cursor lines J and K, so as to adjust the first and second target points, making it very convenient. Of course, the position of each target point has been changed, and the displayed pressure value has also been updated accordingly.

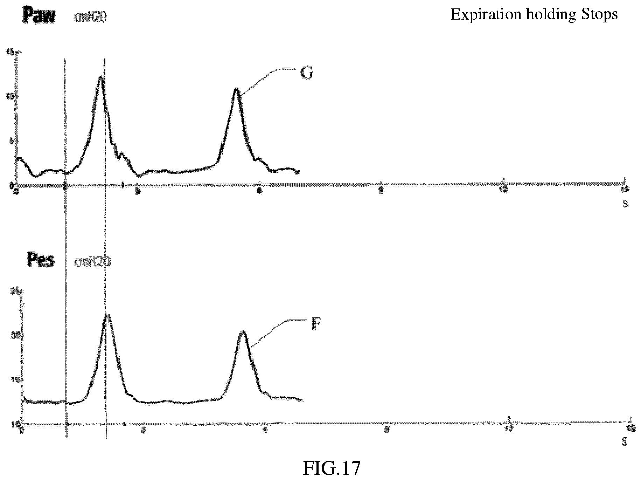

When marking the "peak" points of the airway pressure and the esophageal pressure simultaneously with the second cursor line K, due to potential time delay of the respective peak points of the airway pressure and the esophageal pressure, the second cursor line K can be marked at the "peak" point of the esophageal pressure, as shown in FIG. 15, or can also be marked at the "peak" point of the airway pressure, as shown in FIG. 16, as well as possible not to be marked at either peak point of esophageal or airway pressure. For example, the second cursor line K can be marked at a position between the two peak points, or at a position within a certain range (peak range) of each peak point, as shown in FIG. 17. This marking method can also achieve the ratio calculation of occlusion test. The reason for this marking is that when using one cursor line to simultaneously mark the "peak" points, there may be situations where it is not possible to simultaneously mark the each waveform. Therefore, one can choose the best advantage within a certain range of each peak point to replace it. Furthermore, from another perspective, if the delay time between peak values of esophageal pressure and airway pressure is large, it may lead to inaccurate calculation of the ratio result. The criterion for successful positioning of the balloon for the esophageal pressure is that the ratio result is within 0.8-1.2. Therefore, selecting a better position within a certain range of their respective peak points (the ratio result close to 1) is considered a compensation treatment for this peak delay phenomenon.

Of course, marking the peak value by the cursor line can also be achieved using two cursor lines, one for marking the peak point of the airway pressure and the other for marking the peak point of the esophageal pressure, which is not elaborated here.

In some embodiments, the ratio of the change of the esophageal pressure to the change of the airway pressure can be automatically calculated by the processor 10. Therefore, the above method can also include the following steps.

In step 43, the processor 10 calculates a change of the esophageal pressure Δ Pes according to a pressure value at the first target point of the esophageal pressure and a pressure value at the second target point of the esophageal pressure, calculates a change of the airway pressure Δ Paw according to a pressure value at a first target point of the airway pressure and a pressure value at the second target point of the airway pressure.

In step 44, the processor 10 calculates a ratio of change of the esophageal pressure Δ Pes to the change of the airway pressure Δ Paw and displays the ratio, as shown in FIG. 13, with the displayed ratio of 0.95. The ratio can be Δ Pes/Δ Paw or Δ Paw/Δ Pes. The user can determine whether the pressure measurement apparatus 20 is in the target position range by seeing this ratio. Since the position of each target point is marked in the corresponding trend graph, the user only needs to confirm whether the position of the target point automatically recognized by processor 10 is accurate, and then can determine the accuracy of the ratio calculated by the processor 10, which is very convenient. If the user needs to adjust the position of the target point to make the ratio more accurate, drag its mark (such as a cursor line) to achieve convenient and fast operation.

In step 5', the processor 10 determines whether the ratio is within the preset threshold range, and if so, the process proceeds to step 6'. The specific determination process is the same as step 5 of the embodiment in FIG. 3, and is not elaborated here.

In step 6', when the ratio is within the preset threshold range, the processor 10 outputs second indication information to indicate that the pressure measurement apparatus is already in the target position range, such as displaying the second indication information on the display interface of the display. The details are the same as step 6 of the embodiment in FIG. 3, and is not elaborated here.

In step 7', the processor 10 obtains the pressure data detected by the pressure measurement apparatus in the target position range, and displays it as the target esophageal pressure on the display interface. Thus, the measurement of esophageal pressure is completed, as detailed in step 7 of the embodiment in FIG. 3, and is not further elaborated here.

In summary, in the process of positioning the pressure measurement apparatus, that is the esophageal pressure balloon in this embodiment, the amplitude of change of the pressure during the obstruction test can be automatically identified, and the user can be intelligently indicated to use the characteristic position point (target point) for calculating the amplitude of change of the pressure. The user can confirm that the calculation result for the ratio satisfies expectations through this indication, or adjust the position of the cursor line to generate new ratio result.

Those skilled in the art can recognize that all or part of the functions of various methods in the above embodiments can be achieved through hardware or computer programs. When all or part of the functions in the above embodiments are implemented through a computer program, the program can be stored in a computer-readable storage medium, which can include read-only memory, random access memory, magnetic disk, optical disk, hard disk, etc. The program is executed by a computer to achieve the above functions. For example, storing a program in a device memory and executing the program in the memory through a processor can achieve all or part of the above functions. In addition, when all or part of the functions in the above embodiment are realized by a computer program, the program can also be stored in a storage medium such as a server, another computer, disk, optical disk, USB flash drive or Portable storage device, and saved to the memory of the local device by downloading or copying, or the version of the system of the local device is updated. When the program in the memory is executed by the processor, All or part of the functions in the above implementation can be achieved.

Various exemplary embodiments are described herein. However, those skilled in the art recognizes that changes and modifications may be made to the exemplary embodiments without departing from the scope of this disclosure. For example, the various operation steps and the components configured to perform the operation steps can be implemented in different ways according to the specific application or considering any figure of cost functions associated with the operation of the system (for example, one or more steps can be deleted, modified or combined into other steps).

In addition, as understood by those skilled in the art, the principles herein can be reflected in a computer program product on a computer-readable storage medium which is pre-loaded with computer-readable program code. Any tangible, non-transitory computer-readable storage medium can be used, including magnetic storage devices (hard disk, floppy disk, etc.), optical storage devices (CD-ROM, DVD, Blu ray disk, etc.), flash memory and/or the likes. Computer program instructions can be loaded onto a general-purpose computer, a special-purpose computer, or other programmable data processing device to form a machine, so that these instructions executed on a computer or other programmable data processing device can generate a device to realize a specified function. These computer program instructions may also be stored in a computer-readable memory, which may instruct a computer or other programmable data processing device to operate in a specific manner, so that the instructions stored in the computer-readable memory can form a manufactured article, including an implementation device for realizing a specified function. Computer program instructions can also be loaded on a computer or other programmable data processing device, so that a series of operation steps are performed on the computer or other programmable device to generate a computer implemented process, so that the instructions executed on the computer or other programmable device can provide steps for realizing the specified functions.

Although the principles herein have been shown in various embodiments, many modifications of structures, arrangements, proportions, elements, materials, and components that are particularly applicable to specific environmental and operational requirements may be used without departing from the principles and scope of the present disclosure. The above modifications and other changes or amendments are included in the scope of this disclosure.

The foregoing specific description has been described with reference to various embodiments. However, those skilled in the art can recognize that various amendments and changes may be made without departing from the scope of this disclosure. Accordingly, consideration of this disclosure is illustrative rather than restrictive, and all such modifications are included within its scope. Similarly, there are solutions to the advantages, other advantages, and problems of the various embodiments as described above. However, the benefits, advantages, solutions to problems and any solution that can produce these elements or make them more explicit should not be interpreted as critical, necessary or necessary. The term "include" and any other variations thereof as used herein are non-exclusive inclusions, so that a process, method, article or device that includes a list of elements includes not only these elements, but also other elements that are not explicitly listed or do not belong to the process, method, system, article or device. In addition, the term "connection" and any other variation thereof as used herein refer to physical connection, electrical connection, magnetic connection, optical connection, communication connection, functional connection and/or any other connection.

Those skilled in the art recognize that many changes can be made to the details of the above embodiments without departing from the basic principles of this disclosure. Therefore, the scope of this disclosure shall be determined according to the following attached claims.

## Claims

1. A position indication method for a pressure measurement apparatus of an esophageal pressure, **characterized in that**, comprising:
detecting a position of the pressure measurement apparatus which moves in an esophagus;
outputting first indication information when the pressure measurement apparatus is detected to be in a target position range, wherein the first indication information is configured to indicate that the pressure measurement apparatus is already in the target position range; and
obtaining pressure data which is detected by the pressure measurement apparatus, which is already in the target position range, and using the pressure data as a target esophageal pressure.

2. The method according to claim 1, **characterized in that**, outputting first indication information, comprises at least one of:
displaying text indication information;
displaying graphical indication information;
displaying indication information for displaying picture(s) or video(s);
emitting sound indication information; and
emitting light indication information.

3. The method according to claim 1, **characterized in that**, further comprising:
obtaining airway pressure data and/or the pressure data which is detected by the pressure measurement apparatus; and
generating and displaying a trend graph for an airway pressure according to the airway pressure data, and/or generating and displaying a trend graph for the esophageal pressure according to the pressure data.

4. The method according to claim 3, **characterized in that**, outputting first indication information, comprises:
distinguishingly displaying, in the trend graph for the airway pressure and/or the trend graph for the esophageal pressure, a corresponding region when the pressure measurement apparatus is already in the target position range.

5. The method according to claim 1, **characterized in that**, outputting first indication information when the pressure measurement apparatus is detected to be in a target position range, comprises:
generating a score value, which reflects the detected position of the pressure measurement apparatus, according to the detected position of the pressure measurement apparatus; and
displaying the score value; or displaying a measurement value bar of the score value and marking a position of the score value on the measurement value bar; or displaying a trend graph for a change of the score value and marking a preset qualified range on the trend graph for the change of the score value, wherein the score value within the qualified range is a score value when the pressure measurement apparatus is already in the target position range.

6. The method according to claim 1, **characterized in that**, detecting a position of the pressure measurement apparatus which moves in an esophagus, comprises:
obtaining the pressure data which is detected by the pressure measurement apparatus, generating a waveform curve for the esophageal pressure, detecting a waveform characteristic of the waveform curve for the esophageal pressure, and determining that the pressure measurement apparatus is already in the target position range when the waveform characteristic detected is a preset characteristic;
acquiring image data at the pressure measurement apparatus, and detecting the position of the pressure measurement apparatus by identifying the image data; or
emitting a sound signal or a light signal at the pressure measurement apparatus, and capturing the position of the pressure measurement apparatus by detecting the sound signal or the light signal.

7. The method according to claim 1, **characterized in that**, further comprising:
quantitatively detecting the position of the pressure measurement apparatus which is static and in the target position range, so as to obtain a quantitative value before obtaining the pressure data, which is detected by the pressure measurement apparatus, which is already in the target position range; and
outputting second indication information, when the quantitative value is within a preset threshold range.

8. The method according to claim 7, **characterized in that**, further comprising:
displaying, on a display interface, indication information for doctor operation, when the position of the pressure measurement apparatus, which moves in an esophagus, is detected; and/or
displaying, on a display interface, indication information for doctor operation, when the position of the pressure measurement apparatus, which is static and in the target position range, is quantitatively detected.

9. A medical device, **characterized in that**, comprising:
a pressure measurement apparatus, which is configured to detect a pressure in an esophagus, so as to obtain pressure data;
a detection device, which is configured to detect the pressure measurement apparatus which moves in the esophagus, so as to obtain detection data, which is related to a position of the pressure measurement apparatus; and
a processor, which is configured to determine whether the pressure measurement apparatus is in a target position range according to the detection data, and to output first indication information when the pressure measurement apparatus is detected to be in the target position range, wherein the first indication information is configured to indicate that the pressure measurement apparatus is already in the target position range.

10. A medical device, **characterized in that**, comprising:
a pressure measurement apparatus, which is configured to detect a pressure in an esophagus, so as to obtain pressure data; and
a processor, which is configured to determine whether the pressure measurement apparatus is in a target position range according to the pressure data, and to output first indication information when the pressure measurement apparatus is detected to be in the target position range, wherein the first indication information is configured to indicate that the pressure measurement apparatus is already in the target position range.

11. The medical device according to claim 9 or claim 10, **characterized in that**, in order to output first indication information, the processor is specifically configured to:
display, on a display which is connected with the processor, at least one of: text indication information, graphical indication information, indication information for displaying picture(s) or video(s);
emit, through the display, sound indication information; or
emit, through the display, light indication information.

12. The medical device according to claim 9 or 10, **characterized in that**, the processor is further configured to:
obtain airway pressure data and/or the pressure data which is detected by the pressure measurement apparatus; and
generate and display a trend graph for an airway pressure according to the airway pressure data, and/or generate and display a trend graph for an esophageal pressure according to the pressure data.

13. The medical device according to claim 12, **characterized in that**, in order to output first indication information, the processor is specifically configured to:
distinguishingly display, in the trend graph for the airway pressure and/or the trend graph for the esophageal pressure, a corresponding region when the pressure measurement apparatus is in already the target position range.

14. The medical device according to claim 9 or claim 10, **characterized in that**, in order to output first indication information when the pressure measurement apparatus is detected to be in the target position range, the processor is specifically configured to:
generate a score value, which reflects a detected position of the pressure measurement apparatus, according to the detection data or the pressure data; and
display, on a display which is connected with the processor, the score value; or display, on a display which is connected with the processor, a measurement value bar of the score value and mark a position of the score value on the measurement value bar; or display, on a display which is connected with the processor, a trend graph for a change of the score value and mark a preset qualified range on the trend graph for the change of the score value, wherein the score value within the qualified range is a score value when the pressure measurement apparatus is already in the target position range.

15. The medical device according to claim 9, **characterized in that**, in order to determine whether the pressure measurement apparatus is in a target position range according to the detection data, the processor is specifically configured to:
identify image data to detect the position of the pressure measurement apparatus, when the detection data comprises the image data;
capture the position of the pressure measurement apparatus according to the detection data, when the medical device further comprises a sound generation apparatus which emits a sound signal at the pressure measurement apparatus and the detection device detects the sound signal to obtain the detection data; or
capture the position of the pressure measurement apparatus according to the detection data, when the medical device further comprises a light source which emits a light signal at the pressure measurement apparatus, and the detection device detects the light signal to obtain the detection data.

16. The medical device according to claim 10, **characterized in that**, in order to determine whether the pressure measurement apparatus is in a target position range according to the pressure data, the processor is specifically configured to:
generate a waveform curve for an esophageal pressure according to the pressure data which is detected by the pressure measurement apparatus;
detect a waveform characteristic of the waveform curve for the esophageal pressure; and
determine that the pressure measurement apparatus is in the target position range, when the waveform characteristic detected is a preset characteristic.

17. The medical device according to claim 9 or claim 10, **characterized in that**, the processor is further configured to:
quantitatively detect the position of the pressure measurement apparatus which is static and in the target position range, so as to obtain a quantitative value; and
output second indication information, when the quantitative value is within a preset threshold range.

18. The medical device according to claim 17, **characterized in that**, the processor is further configured to:
display, on a display which is connected with the processor, indication information for doctor operation, when detecting the position of the pressure measurement apparatus, which moves in the esophagus; and/or
display, on a display which is connected with the processor, indication information for doctor operation, when quantitatively detecting the position of the pressure measurement apparatus, which is static and in the target position range.

19. The medical device according to claim 9 or claim 10, **characterized in that**, the medical device is a ventilator, an anesthesia machine or a medical device for measuring an esophageal pressure.

20. A position indication method for a pressure measurement apparatus of an esophageal pressure, **characterized in that**, comprising:
obtaining esophageal pressure data, which is measured by the pressure measurement apparatus, for at least one pressure change cycle;
obtaining airway pressure data for the at least one pressure change cycle;
displaying a pressure comparison view according to the esophageal pressure data and the airway pressure data, wherein the pressure comparison view comprises a trend graph for the esophageal pressure and a trend graph for an airway pressure; and
further displaying, when the pressure comparison view is displayed, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; wherein the first target point of the esophageal pressure and the second target point of the esophageal pressure are two points for calculating a change of the esophageal pressure, and the first target point of the airway pressure and the second target point of the airway pressure are two points for calculating a change of the airway pressure.

21. The method according to claim 20, **characterized in that**, further comprising:
calculating the change of the esophageal pressure according to the pressure value of the first target point of the esophageal pressure and the pressure value of the second target point of the esophageal pressure;
calculating the change of the airway pressure according to the pressure value of the first target point of the airway pressure and the pressure value of the second target point of the airway pressure; and
calculating a ratio of the change of the esophageal pressure to the change of the airway pressure, and displaying the ratio.

22. The method according to claim 21, **characterized in that**, further comprising:
determining whether the ratio is within a preset threshold range; and
outputting second indication information to indicate that the pressure measurement apparatus is already in the target position range, when the ratio is within the preset threshold range.

23. The method according to claim 20, **characterized in that**, the trend graph for the esophageal pressure comprises a curve graph, a scatter graph, a broken line graph, a histogram, a bar graph, a box plot, or a combination thereof;
the trend graph for the airway pressure comprises a curve graph, a scatter graph, a broken line graph, a histogram, a bar graph, a box plot, or a combination thereof.

24. The method according to claim 20, **characterized in that**, the trend graph for the esophageal pressure comprises a waveform curve for the esophageal pressure, and the trend graph for the airway pressure comprises a waveform curve for the airway pressure;
further displaying, when the pressure comparison view is displayed, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; comprises:
on the waveform curve for the esophageal pressure, marking the first target point of the esophageal pressure and the second target point of the esophageal pressure, and displaying the pressure value of the first target point of the esophageal pressure and the pressure value of the second target point of the esophageal pressure; and
on the waveform curve for the airway pressure, marking the first target point of the airway pressure and the second target point of the airway pressure, and displaying the pressure value of the first target point of the airway pressure and the pressure value of the second target point of the airway pressure.

25. The method according to claim 24, **characterized in that**, on the waveform curve for the esophageal pressure, marking the first target point of the esophageal pressure and the second target point of the esophageal pressure, and on the waveform curve for the airway pressure, marking the first target point of the airway pressure and the second target point of the airway pressure, comprise:
simultaneously marking, with a first cursor line, the first target point of the esophageal pressure and the first target point of the airway pressure, and/or simultaneously marking, with a second cursor line, the second target point of the esophageal pressure and the second target point of the airway pressure; when time coordinates of the waveform curve for the esophageal pressure and the waveform curve for the airway pressure are the same, and the waveform curve for the esophageal pressure and the waveform curve for the airway pressure are arranged perpendicularly.

26. The method according to any one of claims 20-25, **characterized in that**, the first target point of the esophageal pressure is a point in an initial range of the change of the esophageal pressure, and the second target point of the esophageal pressure is a point in a peak range of the change of the esophageal pressure;
the first target point of the airway pressure is a point in an initial range of the change of the airway pressure, and the second target point of the airway pressure is a point in a peak range of the change of the airway pressure.

27. A medical device, **characterized in that**, comprising:
a processor, which is configured to:
obtain esophageal pressure data, which is measured by a pressure measurement apparatus, for at least one pressure change cycle;
obtain airway pressure data for the at least one pressure change cycle;
display, on a display, a pressure comparison view according to the esophageal pressure data and the airway pressure data, wherein the pressure comparison view comprises a trend graph for an esophageal pressure and a trend graph for an airway pressure; and
when the pressure comparison view is displayed, further display, through the display, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; wherein the first target point of the esophageal pressure and the second target point of the esophageal pressure are two points for calculating a change of the esophageal pressure, and the first target point of the airway pressure and the second target point of the airway pressure are two points for calculating a change of the airway pressure.

28. The medical device according to claim 27, **characterized in that**, the processor is further configured to:
calculate the change of the esophageal pressure according to the pressure value of the first target point of the esophageal pressure and the pressure value of the second target point of the esophageal pressure;
calculate the change of the airway pressure according to the pressure value of the first target point of the airway pressure and the pressure value of the second target point of the airway pressure; and
calculate a ratio of the change of the esophageal pressure to the change of the airway pressure, and display the ratio.

29. The medical device according to claim 28, **characterized in that**, the processor is further configured to:
determine whether the ratio is within a preset threshold range; and
output second indication information to indicate that the pressure measurement apparatus is already in the target position range, when the ratio is within the preset threshold range.

30. The medical device according to claim 27, **characterized in that**, the trend graph for the esophageal pressure comprises: a curve graph, a scatter graph, a broken line graph, a histogram, a bar graph, and a box plot, or a combination thereof;
the trend graph for the airway pressure comprises a curve graph, a scatter graph, a broken line graph, a histogram, a bar graph, and a box plot, or a combination thereof.

31. The medical device according to claim 27, **characterized in that**, the trend graph for the esophageal pressure comprises a waveform curve for the esophageal pressure, and the trend graph for the airway pressure comprises a waveform curve for the airway pressure;
when the pressure comparison view is displayed, in order to further display, through the display, a time point and/or a pressure value of a first target point of the esophageal pressure, a time point and/or a pressure value of a second target point of the esophageal pressure, a time point and/or a pressure value of a first target point of the airway pressure, a time point and/or a pressure value of a second target point of the airway pressure; the processor is specifically configured to:
on the waveform curve for the esophageal pressure, mark the first target point of the esophageal pressure and the second target point of the esophageal pressure, and display the pressure value of the first target point of the esophageal pressure and the pressure value of the second target point of the esophageal pressure; and
on the waveform curve for the airway pressure, mark the first target point of the airway pressure and the second target point of the airway pressure, and display the pressure value of the first target point of the airway pressure and the pressure value of the second target point of the airway pressure.

32. The medical device according to claim 31, **characterized in that**, in order to on the waveform curve for the esophageal pressure, mark the first target point of the esophageal pressure and the second target point of the esophageal pressure, and on the waveform curve for the airway pressure, mark the first target point of the airway pressure and the second target point of the airway pressure, the processor is specifically configured to:
simultaneously mark, with a first cursor line, the first target point of the esophageal pressure and the first target point of the airway pressure, and/or simultaneously mark, with a second cursor line, the second target point of the esophageal pressure and the second target point of the airway pressure; when time coordinates of the waveform curve for the esophageal pressure and the waveform curve for the airway pressure are the same, and the waveform curve for the esophageal pressure and the waveform curve for the airway pressure are arranged perpendicularly.

33. The medical device according to any one of claims 27-32, **characterized in that**, the first target point of the esophageal pressure is a point in an initial range of the change of the esophageal pressure, and the second target point of the esophageal pressure is a point in a peak range of the change of the esophageal pressure;
the first target point of the airway pressure is a point in an initial range of the change of the airway pressure, and the second target point of the airway pressure is a point in a peak range of the change of the airway pressure.

34. A computer-readable storage medium which comprises a program, **characterized in that**, when the program is executed by a processor, the method according to any one of claims 1-8 is implemented.
